# EUROPÄISCHE PATENTSCHRIFT

(11) **EP 3 071 542 B1**
(45) Veröffentlichungstag und Bekanntmachung des Hinweises auf die Patenterteilung: **27.12.2017**
(21) Anmeldenummer: 14792800.6
(22) Anmeldetag: 31.10.2014
(51) Int. Cl.: C07C 45/50, C07C 47/02

(54) **VERFAHREN ZUR HYDROFORMYLIERUNG VON OLEFINEN**
METHOD FOR THE HYDROFORMYLATION OF OLEFINS
PROCÉDÉ D'HYDROFORMYLATION D'OLÉFINES

(30) Priorität: 18.11.2013 EP 13193266
(43) Veröffentlichungstag der Anmeldung: 28.09.2016
(73) Patentinhaber: BASF SE, 67056 Ludwigshafen am Rhein (DE)
(72) Erfinder: PAPP, Rainer, 67346 Speyer (DE); RUDOLPH, Jens, 67056 Ludwigshafen (DE); THELEN, Hans-Günter, 68259 Mannheim (DE); DIEHL, Klaus, Deer Park, Texas 77536 (US)
(74) Vertreter: BASF IP Association
(86) Internationale Anmeldenummer: PCT/EP2014/073412
(87) Internationale Veröffentlichungsnummer: WO 2015/071113

(56) Entgegenhaltungen:
- EP-A1- 0 850 905
- EP-B1- 1 204 624
- WO-A1-2009/080396

## Beschreibung

Die vorliegende Erfindung betrifft ein Verfahren zur Hydroformylierung von Olefinen mit 6 bis 20 Kohlenstoffatomen.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen die Carbonylkomplexe von Metallen der VIII. Nebengruppe, insbesondere Co, Rh, Ir, Pd, Pt oder Ru eingesetzt, die unmodifiziert oder z. B. mit amin- oder phosphinhaltigen Liganden modifiziert sein können. Eine zusammenfassende Darstellung der großtechnisch ausgeübten Verfahren findet sich bei J. Falbe, "New Syntheses with Carbon Monoxide", Springer Verlag 1980, S. 162 ff.

Während kurzkettige Olefine mit bis zu 5 Kohlenstoffatomen gegenwärtig überwiegend mit ligandenmodifizierten Rhodiumcarbonylen als Katalysator hydroformyliert werden, behauptet bei den längerkettigen Olefinen Kobalt als katalytisch wirksames Zentralatom seine dominierende Stellung. Dies liegt zum einen an der hohen katalytischen Aktivität des Kobaltcarbonylkatalysators unabhängig von der Lage der olefinischen Doppelbindungen, der Verzweigungsstruktur und der Reinheit des umzusetzenden Olefins. Zum anderen kann der Kobaltkatalysator von den Hydroformylierungsprodukten vergleichsweise leicht abgetrennt und in die Hydroformylierungsreaktion zurückgeführt werden. Außerdem können Katalysatorverluste bei der Aufarbeitung aufgrund des geringeren Preises von Kobalt leichter toleriert werden.

In einem üblichen Verfahren zur Abtrennung und Rückführung des Kobaltkatalysators wird die organische Phase des Reaktoraustrags von den Kobaltcarbonylkomplexen durch Behandlung mit Sauerstoff oder Luft in Gegenwart von schwach saurem Wasser befreit (vgl. DE-AS 24 04 855). Dabei wird der Kobaltkatalysator oxidativ zerstört und das Zentralatom formal von der Oxidationsstufe -1 nach +2 überführt und kann sodann durch Extraktion mit der wässrigen Lösung entfernt werden (Entkobaltung). Aus der wässrigen Kobalt(II)-salzlösung kann durch Umsetzen mit Kohlenmonoxid und Wasserstoff wieder der für die Hydroformylierung benötigte Katalysatorkomplex hergestellt werden (Carbonylbildung). Der hergestellte Kobaltkatalysator wird dann aus der wässrigen Phase mit einer organischen Phase, vorzugsweise dem zu hydroformylierenden Olefin, extrahiert (Katalysatorextraktion). Es können auch neben dem Olefin die Reaktions- und Nebenprodukte der Hydroformylierung zur Katalysatorextraktion eingesetzt werden. Die mit dem Kobaltkatalysator beladenen Olefine werden dann unter erhöhtem Druck und erhöhter Temperatur in einem Reaktor hydroformyliert (Olefinhydroformylierung).

Der Reaktionsaustrag wird, insbesondere bei Verwendung eines vertikalen Reaktorrohres, üblicherweise am Kopf des Reaktors entnommen. Insbesondere bei höheren Olefinen mit 8 Kohlenstoffatomen oder mehr wird die der Reaktionszone zugeführte wässrige Phase, die zur Erreichung einer ausreichenden Katalysatorkonzentration in der Reaktionszone notwendig ist, nicht vollständig in gelöster oder suspendierter Form mit dem über Kopf abgenommenen Reaktionsgemisch abgeführt. Deshalb wird Reaktionsaustrag vorzugsweise nicht nur am Kopf, sondern auch aus dem Sumpfraum entnommen.

Die ungeregelte oder nicht ausreichend geregelte Entnahme wässriger Phase aus dem Sumpfraum führt zu Betriebsstörungen. Bei einem Überschuss wässriger Phase kann es zu einer Verringerung des Umsatzes kommen. Ein Unterschuss wässriger Phase kann zu lokalen Temperaturspitzen führen, die eine Zersetzung des Kobaltkatalysators verursachen.

Im Stand der Technik wird die Menge des aus dem Sumpfraum entnommenen Reaktionsaustrags nach Maßgabe des Stands der wässrigen Sumpfphase im Reaktor bestimmt.

Die EP 1 204 624 B1 offenbart ein kontinuierliches Verfahren zur Hydroformylierung von Olefinen mit 6 bis 20 Kohlenstoffatomen, bei dem eine einen Kobaltkatalysator enthaltende wässrige Phase in wenigstens einer Reaktionszone (z.B. in einem vertikalen Rohrreaktor) mit Olefinen sowie Wasserstoff und Kohlenmonoxid innig in Kontakt gebracht wird. Reaktionsaustrag kann sowohl am Kopf des Reaktors als auch aus dem Sumpfraum des Reaktors entnommen werden. Die Entnahme des Reaktionsaustrags aus dem Sumpfraum erfolgt vorzugsweise phasengeregelt.

Die EP 1 279 658 B1 offenbart ein Verfahren zur Hydroformylierung von Olefinen mit 5 bis 24 Kohlenstoffatomen zu den entsprechenden Aldehyden und/oder Alkoholen mit 6 bis 25 Kohlenstoffatomen in Gegenwart von unmodifizierten Kobaltkatalysatoren in einem Einstufenprozess, wobei die wässrige Sumpfphase im Reaktor mit der organischen Phase durchmischt wird, die Konzentration an Kobaltverbindungen in der wässrigen Sumpfphase im Bereich von 0,4 bis 1,7 Massen-% liegt und der Stand der wässrigen Sumpfphase im Reaktor im stationären Zustand konstant gehalten wird.

Jedoch ist der Stand der wässrigen Sumpfphase unter den im Reaktor herrschenden Bedingungen oft nicht exakt zu bestimmen.

Die radiometrische Messung des Stands der wässrigen Sumpfphase ist ungenau. Die γ-Strahlung wird bei der Passage der 30 cm starken Stahlwände abgeschwächt. Außerdem ist die Kalibrierung der radiometrische Messung schwierig, da man den im Reaktor vorliegenden Stand der wässrigen Sumpfphase in der Regel auch bei der Kalibrierung nicht genau kennt.

Ferner erfordert die Instandhaltung von Vorrichtungen zur Bestimmung des Stands der wässrigen Sumpfphase einen großen Aufwand, wenn sie im Reaktor angeordnet und demzufolge nur mit großem Aufwand unter Unterbrechung des Verfahrens zugänglich sind.

Die ungenaue Bestimmung des Stands der wässrigen Sumpfphase führt zu einer nicht ausreichend genau regelbaren Entnahme wässriger Phase aus dem Sumpfraum und damit zu Betriebsstörungen, die letztlich auch die Ausbeute rohen Hydroformylierungsproduktes verringern.

Der vorliegenden Erfindung liegt die Aufgabe zugrunde, ein Verfahren zur Hydroformylierung von Olefinen mit 6 bis 20 Kohlenstoffatomen bereitzustellen, bei dem die Ausbeute rohen Hydroformylierungsproduktes in Folge stabilen Dauerbetriebs erhöht ist.

Die Aufgabe wird gelöst durch ein kontinuierliches Verfahren zur Hydroformylierung von Olefinen mit 6 bis 20 Kohlenstoffatomen in Gegenwart eines Kobaltkatalysators in Gegenwart einer wässrigen Phase unter Durchmischung in einem Reaktor, wobei man einen Hydroformylierungsprodukte enthaltenden ersten Strom am Kopf des Reaktors und einen wässrige Phase enthaltenden zweiten Strom aus dem Sumpf des Reaktors abzieht, das dadurch gekennzeichnet ist, dass die Flussrate des zweiten Stroms nach Maßgabe einer Temperatur geregelt wird, die an einer Stelle im Sumpf des Reaktors oder in einer aus dem Sumpf führenden Leitung gemessen wird.

Wenn sich wässrige Phase von der organischen Phase entmischt und im Sumpfraum des Reaktors abscheidet, wird sie nicht mehr mit zu hydroformylierenden Olefinen gemischt. Die Reaktionsrate im Sumpfraum des Reaktors sinkt und die abgeschiedene wässrige Phase kühlt sich langsam ab. Es wurde gefunden, dass aus dem Sumpfraum des Reaktors ein Strom temperaturgeregelt abgezogen werden kann. Damit wird verhindert, dass die wässrige Phase zu hoch steigt. Die Regelung kann auch automatisiert erfolgen.

Die Temperatur an der Stelle im Sumpf des Reaktors oder in der aus dem Sumpf führenden Leitung lässt sich mit geringem Aufwand exakt bestimmen. Auch der Instandhaltungsaufwand ist gering. Demzufolge ist die Zahl der durch die Instandhaltung der Temperaturmessvorrichtung bedingten Unterbrechungen des Verfahrens gering. Mit dem Verfahren wird ein stabiler Dauerbetrieb erreicht.

Die Flussrate des zweiten Stroms nach Maßgabe einer Temperatur zu regeln, die an der Stelle im Sumpf des Reaktors oder in der aus dem Sumpf führenden Leitung gemessen wird, bedeutet, dass die an der Stelle im Sumpf des Reaktors oder in der aus dem Sumpf führenden Leitung gemessene Temperatur als eine Regelgröße in die Regelung der Flussrate des zweiten Stroms eingeht. Daneben können weitere Regelgrößen erhoben werden und in die Regelung eingehen. Die Regelung kann durch eine computerbasierte Prozesssteuerung erfolgen. In einer Steuereinheit kann der Einfluss der Änderung einer Stellgröße auf eine oder mehrere Regelgrößen als mathematisches Modell bzw. Algorithmus hinterlegt werden. Aus den gemessenen Werten einer oder mehrerer Regelgrößen werden Stelleingriffe zur Regelung ermittelt. Geeignete Modelle und Programme, die zur Implementierung der vorliegenden Erfindung Anwendung finden können, sind dem Fachmann geläufig. Im einfachsten Fall erfolgt die Regelung manuell, indem der Operateur bei einer Veränderung der Regelgröße entsprechende Stellgrößen anpasst.

Misst man diese Temperatur im Sumpf des Reaktors, so kann die Messung an einer Stelle erfolgen, an der sich die wässrige Phase als spezifisch schwerere Phase ansammelt. Der Innenraum des Reaktors ist durch Boden, Deckel und Außenwand abgegrenzt. Die Höhe des Innenraums erstreckt sich vom tiefsten zum höchsten Punkt des Innenraums. Bezogen auf die Höhe des Innenraums kann die Temperaturmessung im Sumpf beispielsweise im untersten Zwanzigstel, bevorzugt im untersten Fünfzigstel des Innenraums erfolgen. Vorzugsweise erfolgt die Temperaturmessung an einer Stelle unterhalb der Mündung einer Zufuhrleitung, durch die man das Olefin und/oder andere Reaktanden zuführt, z.B. nahe am Abzug des wässrige Phase enthaltenden zweiten Stroms.

Vorzugsweise misst man diese Temperatur jedoch in einer aus dem Sumpf führenden Leitung. Bei der Leitung handelt es sich vorzugsweise um eine Leitung, durch die man den zweiten Strom aus dem Sumpf des Reaktors abzieht. Grundsätzlich kommt als die Leitung, in der man die Temperatur misst, jede Leitung in Betracht, die ein Maß für die im Sumpf des Reaktors vorherrschende Temperatur bietet.

In einer bevorzugten Ausführungsform wird die Flussrate des zweiten Stroms nach Maßgabe der Differenz zwischen der Reaktionstemperatur und der an der Stelle im Sumpf des Reaktors oder in der aus dem Sumpf führenden Leitung gemessenen Temperatur geregelt. Die Reaktionstemperatur wird geeigneter Weise an wenigstens einer Stelle intensiver Durchmischung im Reaktor gemessen. Vorzugsweise wird die Reaktionstemperatur an mehreren Stellen intensiver Durchmischung im Reaktor gemessenen, z.B. an 2, 3, 4, 5 oder 6 Messpunkten, und gemittelt. Geeignete Stellen zur Messung der Reaktionstemperatur sind z.B. die Eintrittsorte von Olefin oder Oxogas in den Reaktor, Positionen in der unmittelbaren Umgebung von Mischorganen im Reaktor oder in der Nähe von Einbauten im Reaktor, die der intensiven Durchmischung des Reaktorinhalts dienen (z.B. Stromstörer).

Man kann die Flussrate des aus dem Sumpf des Reaktors abgezogenen zweiten Stroms erhöhen, wenn die maßgebliche Temperaturdifferenz zunimmt. Beispielsweise kann man die Flussrate des aus dem Sumpf des Reaktors abgezogenen zweiten Stroms erhöhen, wenn die maßgebliche Temperaturdifferenz einen ersten Schwellenwert überschreitet und verringern, wenn die maßgebliche Temperaturdifferenz einen zweiten Schwellenwert unterschreitet. Der zweite Schwellenwert kann um 0 bis 5 °C niedriger sein, als der erste Schwellenwert. D. h., dass beide Schwellenwerte auch gleich sein können. Vorzugsweise sind beide Schwellenwerte aus einem Bereich von 1 bis 10 °C, bevorzugt 1 bis 7 °C, insbesondere bevorzugt 2 bis 5°C ausgewählt.

Vorzugsweise zieht man den zweiten Strom aus dem Sumpf des Reaktors ab, indem man ihn durch eine regelbare Vorrichtung aus dem Sumpf des Reaktors führt. Bei der Vorrichtung handelt es sich vorzugsweise um ein Ventil. Vorzugsweise öffnet man das Ventil, sobald der erste Schwellenwert überschritten wird und schließt das Ventil, sobald der zweite Schwellenwert unterschritten wird. Man regelt das Ventil vorzugsweise automatisch über eine geeignete Regelungseinheit. Die Regelungseinheit berechnet die Temperaturdifferenz und überträgt ein von der Temperaturdifferenz abhängiges Signal zum Öffnen oder Schließen an das Ventil.

Geeignete, druckfeste Reaktoren zur Durchführung des erfindungsgemäßen Verfahrens sind dem Fachmann bekannt. Dazu zählen die allgemein üblichen Reaktoren für Gas-flüssig-Reaktionen, wie z. B. Rohrreaktoren, Rührkessel, Gasumlaufreaktoren, Blasensäulen, Schlaufenreaktoren usw., die gegebenenfalls durch Einbauten nochmals unterteilt sein können. Geeignet ist beispielsweise ein Schlaufenreaktor, ein vertikaler Hochdruck-Blasensäulenreaktor, der gegebenenfalls mit koaxialen rohrförmigen Einbauten versehen ist, oder ein vertikaler Rohrreaktor. Vorzugsweise ist der Reaktor ein vertikaler Blasensäulenreaktor oder ein vertikaler Rohrreaktor.

Die Temperatur im Reaktor liegt im Allgemeinen bei 100 bis 250 °C, insbesondere 145 bis 200 °C. Der im Reaktor vorherrschende Druck liegt vorzugsweise im Bereich von 100 bis 400 bar, insbesondere 200 bis 300 bar.

Man kann zur Variation der Durchmischung einen Teil des im Reaktor enthaltenen Reaktionsgemischs im oberen Bereich des Reaktors ausleiten und zurück in den unteren Bereich des Reaktors führen. Beispielsweise kann man das ausgeleitete Reaktionsgemisch über eine Pumpe führen und anschließend mit den Ausgangsstoffen vereinigen und die mit dem ausgeleiteten Reaktionsgemisch vereinigten Ausgangsstoffe in den Reaktor führen.

Mit dem Begriff "Olefine" ist vorliegend sowohl ein Olefin, wie auch Mischungen verschiedener, z. B. isomerer Olefine, bezeichnet. Nach dem erfindungsgemäßen Verfahren lassen sich Olefine mit 6 bis 20 Kohlenstoffatomen hydroformylieren. Das erfindungsgemäße Verfahren ist insbesondere für die Hydroformylierung von isomeren Olefingemischen geeignet, die durch Oligomerisierung von niederen Olefinen, wie Propen und Butenen, hergestellt werden. Zu den typischen Oligomerisaten, die als Olefine für das vorliegende Verfahren geeignet sind, zählen unter anderem Di-, Tri- und Tetrapropen, Di-, Tri- und Tetrabuten sowie Mischoligomerisate von Propenen und Butenen. Die Oligomerisate von Butenen sind nach bekannten Oligomerisierungsverfahren, z. B. nach dem Octol-Prozess von Hüls und dem Dimersol-Verfahren von IFP, großtechnisch zugänglich. Bevorzugt werden Oligomere auf der Basis von sogenanntem Raffinat 2 eingesetzt, einem Strom, der im wesentlichen lineare Butene, n- und iso-Butan sowie nicht mehr als 5 Gew.-% Isobuten, bevorzugt nicht mehr als 3 Gew.-% Isobuten enthält. Nach dem erfindungsgemäßen Verfahren können ferner lineare langkettige Olefine mit endständiger Doppelbindung, die z. B. nach dem SHOP-Prozess oder Ziegler-Verfahren erhältlich sind, oder lineare langkettige Olefine mit innenliegender Doppelbindung hydroformyliert werden.

Die Hydroformylierung erfolgt in Gegenwart von Wasserstoff und Kohlenmonoxid. Die beiden Gase werden üblicherweise in Form eines Gemisches, dem sogenannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel etwa 10 : 1 bis 1 : 10, insbesondere 2,5 : 1 bis 1 : 2,5. Ein bevorzugtes Verhältnis liegt bei etwa 40 : 60 (CO : H₂).

Beim erfindungsgemäßen Verfahren erfolgen die Katalysatorextraktion und Olefinhydroformylierung in einem Schritt im Reaktor. Die Carbonylbildung kann gleichzeitig damit oder in einer optionalen vorgelagerten Katalysatorbildungsstufe (Vorcarbonylierung) erfolgen.

In der optionalen Katalysatorbildungsstufe (Vorcarbonylierung) bringt man eine wässrige Kobalt(II)-salzlösung mit Kohlenmonoxid und Wasserstoff unter Bildung des Kobaltkatalysators innig in Kontakt. Dies erfolgt in Anwesenheit oder Abwesenheit der zu hydroformylierenden Olefine und vorzugsweise bei Temperaturen von 50 bis 200 °C, insbesondere 60 bis 140 °C und unter Drücken von 100 bis 400 bar, insbesondere 200 bis 300 bar. Die Katalysatorbildung erfolgt bevorzugt in üblichen Apparaten für Gas-flüssig-Reaktionen, wie Rührbehälter mit Begasungsrührer, Blasensäulen oder Rieselbettkolonnen. Mit Vorteil setzt man in der Katalysatorbildungsstufe Aktivkohle, Zeolithe oder basische Ionenaustauscher ein, die mit Kobaltcarbonyl beladen sind, gemäß Beschreibung in der DE-OS 21 39 630. Die in der Katalysatorbildungsstufe erhaltene, Kobalt(II)-salze und Kobaltkatalysator enthaltende wässrige Lösung wird dann zusammen mit den zu hydroformylierenden Olefinen und gegebenenfalls mitverwendeten organischen Lösungsmitteln sowie Wasserstoff und Kohlenmonoxid in den Reaktor geleitet.

Die Carbonylbildung führt man mit besonderem Vorteil in der vorgelagerten Katalysatorbildungsstufe durch, wenn lineare längerkettiger Olefine mit endständiger Doppelbindung zu vorwiegend geradkettigen Aldehyden/Alkoholen hydroformyliert werden. Um die Bildung unerwünschter verzweigter Hydroformylierungsprodukte zu minimieren, werden im Reaktor dann in der Regel niedrigere Reaktionstemperaturen angewandt. Unter den dann im Reaktor angewandten Bedingungen bildet sich der aktive Kobaltkatalysator jedoch nicht mit genügender Geschwindigkeit.

In vielen Fällen ist es im Hinblick auf den geringeren verfahrenstechnischen Aufwand bevorzugt, dass die Carbonylbildung, die Katalysatorextraktion und die Olefinhydroformylierung einhergehend miteinander im selben Reaktor erfolgen. In einer bevorzugten Ausführungsform des Verfahrens erfolgt die Bildung des Kobaltkatalysators, die Extraktion des Kobaltkatalysators in die organische Phase und die Hydroformylierung der Olefine einhergehend miteinander im selben Reaktor indem die wässrige Kobalt(II)-salzlösung, die Olefine und gegebenenfalls organische Lösungsmittel in der Reaktionszone unter Hydroformylierungsbedingungen innig in Kontakt gebracht werden.

Unabhängig davon, ob die Carbonylbildung in einer vorgelagerten Katalysatorbildungsstufe erfolgt, oder nicht, werden die Ausgangsstoffe vorzugsweise so in den Reaktor eingebracht, dass eine gute Phasenvermischung erfolgt und eine möglichst hohe Phasenaustauschfläche erzeugt wird. Hierfür können die dem Fachmann bekannten Dosiervorrichtungen, wie z. B. mit Füllkörpern befüllte Turbulenzrohre oder Mischdüsen für Mehrphasensysteme eingesetzt werden. Das Durchmischen erfolgt besonders bevorzugt mit einer Mischdüse unter Aufrechterhaltung einer turbulenten Strömung im Reaktor. So erfolgt in einer geeigneten Ausführungsform das Durchmischen, indem die wässrige Kobalt(II)-salzlösung, Olefine sowie Kohlenmonoxid und Wasserstoff mittels einer Mischdüse gleichzeitig in ein Umlaufsystem, wie in den DE-AS 12 05 514 und 19 38 102 beschrieben, in den Reaktor eingebracht werden. Das gegebenenfalls mitverwendete organische Lösungsmittel liegt entweder im Reaktor vor oder wird gleichzeitig mit den anderen Ausgangsstoffen, insbesondere mittels einer Mischdüse, in den Reaktor eingebracht.

Als gegebenenfalls mitzuverwendende organische Lösungsmittel kommen inerte Kohlenwasserstoffe, wie Paraffinfraktionen, aromatische Kohlenwasserstoffe, wie Benzol, Toluol oder Xylol, oder ein Aldehyd und/oder Alkohol, insbesondere aber das Hydroformylierungsprodukt des eingesetzten Olefins, in Betracht. Es können ferner hochsiedende Nebenprodukte der Hydroformylierung als Lösungsmittel verwendet werden. Die Mitverwendung eines Lösungsmittels kann z. B. zur Viskositätserniedrigung bei der Hydroformylierung von langkettigen Olefinen zu langkettigen Aldehyden vorteilhaft sein.

Der am Kopf des Reaktors abgezogene erste Strom enthält neben Hydroformylierungsprodukten auch nicht umgesetzte Olefine und wesentliche Mengen wässriger Phase. Der aus dem Sumpf des Reaktors abgezogene zweite Strom kann neben wässriger Phase wesentliche Mengen an teilumgesetzter organischer Phase enthalten. Vorzugsweise enthält der aus dem Sumpf des Reaktors abgezogene zweite Strom 10 bis 80 Vol.-% wässrige Phase.

Der beschriebene Reaktor weist eine sogenannte Rührkesselcharakteristik auf, es handelt sich um einen gut durchmischten Reaktor. In einem solchen Reaktor ist bei begrenztem Reaktorvolumen nur ein Teilumsatz möglich. Es ist daher im Sinne einer möglichst hohen Wertproduktausbeute bevorzugt, die Hydroformylierung in einem Nachreaktor fortzuführen und möglichst zu vervollständigen. In einer bevorzugten Ausführungsform leitet man den ersten Strom und den zweiten Strom in einen Nachreaktor.

Die Temperatur im Nachreaktor liegt im Allgemeinen bei 100 bis 250 °C, insbesondere 145 bis 200 °C. Der im Nachreaktor vorherrschende Druck liegt vorzugsweise im Bereich von 100 bis 400 bar, insbesondere 200 bis 300 bar. Ein gleichmäßiger Stofftransport vom Reaktor in den Nachreaktor erfolgt vorzugsweise durch Aufrechterhalten eines konstanten Differenzdrucks von wenigen bar zwischen Reaktor und Nachreaktor. Der Differenzdruck beträgt vorzugsweise 2 bis 5 bar. Der Differenzdruck erlaubt eine bequeme Regelung der Ausschleusungen aus dem ersten Reaktor. Der Differenzdruck kann durch ein verstellbares Ventil oder durch eine Blende aufrechterhalten werden.

Vorzugsweise zieht man auch am Nachreaktor einen Hydroformylierungsprodukte enthaltenden ersten Strom am Kopf und einen wässrige Phase enthaltenden zweiten Strom aus dem Sumpf ab. Vorzugsweise wird auch der aus dem Sumpf des Nachreaktors abgezogene zweite Strom temperaturgeregelt abgezogen. Demnach regelt man in einer Ausführungsform die Flussrate des aus dem Nachreaktor abgezogenen zweiten Stroms nach Maßgabe einer Temperatur, die an einer Stelle im Sumpf des Nachreaktors oder in einer aus dem Sumpf des Nachreaktors führenden Leitung gemessen wird. Vorzugsweise nach Maßgabe der Differenz zwischen der Reaktionstemperatur im Nachreaktor und der an der Stelle im Sumpf des Nachreaktors oder in der aus dem Sumpf des Nachreaktors führenden Leitung gemessen Temperatur. Für die Ausschleusung von Strömen aus dem Nachreaktor gelten die vorstehenden Ausführungen zum ersten Reaktor entsprechend. Damit die Ausschleusungen gut geregelt werden können, wird zweckmäßigerweise ein Differenzdruck zwischen dem Nachreaktor und der Austragsleitung eingestellt. Dies kann durch ein verstellbares Ventil oder durch eine Blende erfolgen. Auch dieser Differenzdruck beträgt vorzugsweise 2 bis 5 bar.

In einer bevorzugten Ausführungsform behandelt man den aus dem Reaktor abgezogenen ersten und zweiten Strom, oder bei Verwendung eines Nachreaktors, den aus dem Nachreaktor abgezogenen ersten und zweiten Strom in Gegenwart von wässriger Kobalt(II)-salzlösung mit Sauerstoff, wobei der Kobaltkatalysator unter Bildung von Kobalt(II)-salzen zersetzt wird und diese in die wässrige Phase extrahiert werden; und trennt die Phasen anschließend (Entkobaltung). Die aus dem Reaktor bzw. Nachreaktor abgeführten und gegebenenfalls vereinigten Ströme entspannt man geeigneterweise auf Mitteldruck, in der Regel 10 bis 80 bar, bevorzugt 10 bis 50 bar und behandelt sie anschließend in Gegenwart von wässriger Kobalt(II)-salzlösung mit Sauerstoff. Vorzugsweise stellt man den Sauerstoff bereit, indem man Luft zuführt. Die Behandlung erfolgt vorzugsweise bei Temperaturen von 90 bis 130 °C. Die Kobalt(II)-salzlösung wird aus der nachgeschalteten Phasentrennung zurückgeführt und wird der Entkobaltung bevorzugt mit etwa dem gleichen Massenstrom wie der organische Austrag aus dem (Nach)reaktor zugegeben. Die Kobalt(II)-salzlösung ist vorzugsweise schwach sauer bei einem pH-Wert von 3 bis 5, bevorzugt 3,5 bis 4,5. Man kann die Behandlung in einem mit Füllkörpern, wie z. B. Raschig-Ringen, befüllten Druckbehälter durchführen, in dem eine möglichst hohe Phasenaustauschfläche erzeugt wird oder aber durch Entspannen in einem leeren Druckbehälter. Man trennt die organische Produktphase von der wässrigen Phase anschließend vorzugsweise in einem nachgeschalteten Phasentrenngefäß.

Anschließend kann die nach Abtrennung der wässrigen Phase verbleibende organische Phase geeignet aufgearbeitet, z. B. destilliert und/oder hydriert, werden.

Die wässrige Phase (Kobalt(II)-salzlösung) wird einerseits in den Reaktor oder in die Katalysatorbildungsstufe geführt, andererseits in die Entkobaltung.

Das erfindungsgemäße Verfahren wird durch die beigefügten Figuren näher veranschaulicht.

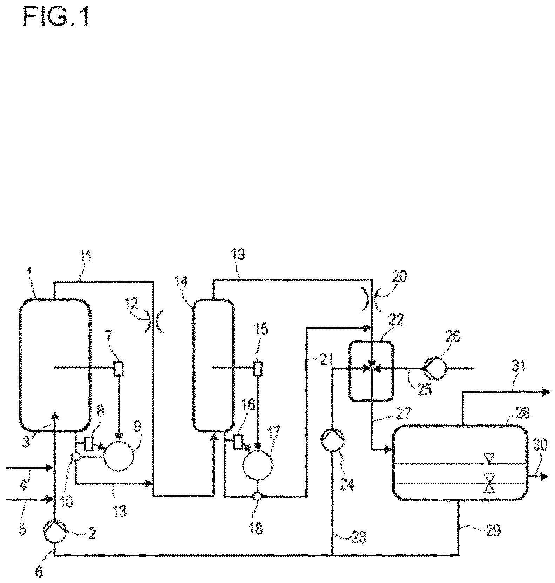

Figur 1 zeigt schematisch eine Anlage zur Durchführung des Verfahrens. Die Anlage umfasst einen Reaktor und einen Nachreaktor, wobei sowohl die Flussrate des aus dem Sumpf des Reaktors, wie auch die Flussrate des aus dem Sumpf des Nachreaktors abgeführten Stroms erfindungsgemäß geregelt wird.

Gemäß Figur 1 wird in einen Reaktor **1** über eine Pumpe **2** und eine Düse **3** Oxogas **4,** Olefin **5** und wässrige Kobalt(II)-salzlösung **6** eingebracht. Die Temperatur an einer Stelle intensiver Durchmischung im Reaktor wird über ein Thermoelement **7** gemessen. Die Temperatur in der aus dem Sumpf des Reaktors führenden Leitung **13** wird über Thermoelement **8** gemessen. Die gemessenen Temperaturen führt man der Regelungseinheit **9** zu. In Regelungseinheit **9** wird eine Temperaturdifferenz berechnet, indem die über Thermoelement **8** gemessene Temperatur von der über Thermoelement **7** gemessenen Temperatur abgezogen wird. Sobald die Temperaturdifferenz einen ersten Schwellenwert überschreitet, geht von der Regelungseinheit **9** ein Signal zum Öffnen des Ventils **10** aus. Sobald die Temperaturdifferenz einen zweiten Schwellenwert unterschreitet, geht von der Regelungseinheit **9** ein Signal zum Schließen des Ventils **10** aus.

Einen Hydroformylierungsprodukte enthaltenden ersten Strom zieht man am Kopf des Reaktors **1** über Leitung **11** ab und führt ihn über die Blende **12.** Einen wässrige Phase enthaltenden zweiten Strom zieht man, sofern Ventil **10** geöffnet ist, aus dem Sumpf des Reaktors ab und leitet ihn über Leitung **13** in Leitung **11.**

Über Leitung **11** bringt man in einen Nachreaktor **14** die vereinigten, am Kopf und aus dem Sumpf des Reaktors abgezogenen Ströme ein. Die Temperatur an einer Stelle intensiver Durchmischung im Nachreaktor wird über Thermoelement **15** gemessen. Die Temperatur in der aus dem Sumpf des Reaktors führenden Leitung **21** wird über Thermoelement **16** gemessen. Die von den Thermoelementen **15** und **16** gemessenen Temperaturen führt man der Regelungseinheit **17** zu. In Regelungseinheit **17** wird eine Temperaturdifferenz berechnet, indem die über Thermoelement **16** gemessene Temperatur vor der über Thermoelement **15** gemessenen Temperatur abgezogen wird. Sobald die Temperaturdifferenz einen ersten Schwellenwert überschreitet, geht von der Regelungseinheit **17** ein Signal zum Öffnen des Ventils **18** aus. Sobald die Temperaturdifferenz einen zweiten Schwellenwert unterschreitet, geht von der Regelungseinheit **17** ein Signal zum Schließen des Ventils **18** aus. Einen Hydroformylierungsprodukte enthaltenden ersten Strom zieht man am Kopf des Nachreaktors **14** über Leitung **19** ab und führt ihn über die Blende **20.** Einen wässrige Phase enthaltenden zweiten Strom zieht man, sofern Ventil **18** geöffnet ist, aus dem Sumpf des Nachreaktors ab und leitet ihn über Leitung **21** in Leitung **19** ein.

Über Leitung **19** bringt man in einem Heißentspannungsbehälter **22**, in dem ein niedrigerer Druck herrscht, als im Reaktor und Nebenreaktor, die vereinigten, am Kopf und aus dem Sumpf des Nachreaktors abgezogenen Ströme ein, wo man sie in Gegenwart von wässriger Kobalt(II)-salzlösung, die über Leitung **23** und Pumpe **24** zugeführt wird, mit Luft, die über Leitung **25** und Kompressor **26** zugeführt wird, behandelt.

Das im Heißentspannungsbehälter anfallende Gemisch führt man über Leitung **27** in den Phasenscheider **28,** aus dem eine wässrige Phase über Leitung **29** ausgeleitet wird. Das rohe Hydroformylierungsprodukt wird über Leitung **30** aus dem Phasenscheider geleitet. Abgas wird über Leitung **31** aus dem Phasenscheider geleitet.

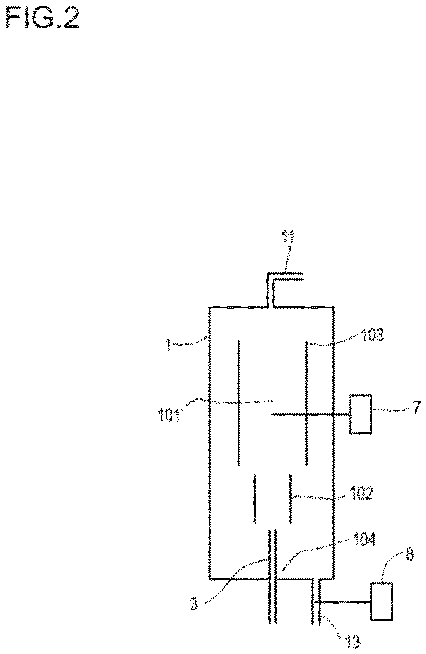

Figur 2 zeigt schematisch einen Reaktor zur Durchführung des erfindungsgemäßen Verfahrens. Dieselben Bezugszeichen haben dieselbe Bedeutung als in Figur 1. Der Reaktor umfasst einen Bereich intensiver Durchmischung **101,** eine Mischzone **102,** eine Nachmischzone **103,** die durch zylindrische Rohre gebildet werden und den Sumpf des Reaktors **104.** Die über Thermoelement **7** gemessene Temperatur wird an einer Stelle im Bereich intensiver Durchmischung gemessen. Die Temperatur in der aus dem Sumpf des Reaktors führenden Leitung **13** wird über Thermoelement **8** gemessen.

### Beispiel

In einem in einer Anlage gemäß Figur 1 betriebenen kontinuierlichen Verfahren wurden folgende Parameter eingestellt:

| | |
|---|---|
| Oxogas (CO:H₂ = 40:60) (**4**) | 3 300 kg/h |
| Isoocten (**5**) | 10 000 kg/h |
| wässrige Kobalt(II)formiatlösung (**6**), ca. 1,2 Gew.-% Cobalt | 1 100 kg/h |
| Reaktionstemperatur im Reaktor (**1**), gemessen durch Thermoelement (**7**) | 187 °C |
| Schwellenwert zur Öffnung und zum Schließen des Ventils (**10**) | 2 °C |
| Über Blende (**12**) eingestellte Druckdifferenz zwischen Leitung (**11**) und Leitung (**13**) | 4 bar |
| Reaktionstemperatur im Nachreaktor (**14**), gemessen durch Thermoelement (**15**) | 187 °C |
| Schwellenwert zur Öffnung und zum Schließen des Ventils (**18**) | 2 °C |
| Durchmesser der Blende (**20**) in Leitung (**19**) | 16 mm |
| wässrige Kobalt(II)-salzlösung (**23**) | 9 000 kg/h |
| Luft (**25**) | 50 kg/h |
| Abgas (**31**) | 500 kg/h |

Die Ausbeute rohen Hydroformylierungsprodukts (**30**) betrug 12 400 kg/h.

## Patentansprüche

1. Kontinuierliches Verfahren zur Hydroformylierung von Olefinen mit 6 bis 20 Kohlenstoffatomen in Gegenwart eines Kobaltkatalysators in Gegenwart einer wässrigen Phase unter Durchmischung in einem Reaktor, wobei man einen Hydroformylierungsprodukte enthaltenden ersten Strom am Kopf des Reaktors und einen wässrige Phase enthaltenden zweiten Strom aus dem Sumpf des Reaktors abzieht, **dadurch gekennzeichnet, dass** die Flussrate des zweiten Stroms nach Maßgabe einer Temperatur geregelt wird, die an einer Stelle im Sumpf des Reaktors oder in einer aus dem Sumpf führenden Leitung gemessen wird.

2. Verfahren nach Anspruch 1, wobei die Flussrate des zweiten Stroms nach Maßgabe der Differenz zwischen einer an wenigstens einer Stelle intensiver Durchmischung im Reaktor gemessenen Reaktionstemperatur und der an der Stelle im Sumpf des Reaktors oder in der aus dem Sumpf führenden Leitung gemessenen Temperatur geregelt wird.

3. Verfahren nach Anspruch 1 oder 2, wobei man die Flussrate des zweiten Stroms erhöht, wenn die Temperaturdifferenz zunimmt.

4. Verfahren nach einem der vorhergehenden Ansprüche, wobei das Durchmischen mittels einer Mischdüse erfolgt.

5. Verfahren nach einem der vorhergehenden Ansprüche, wobei man den ersten Strom und den zweiten Strom in einen Nachreaktor leitet.

6. Verfahren nach Anspruch 5, wobei man einen Hydroformylierungsprodukte enthaltenden ersten Strom am Kopf des Nachreaktors und einen wässrige Phase enthaltenden zweiten Strom aus dem Sumpf des Nachreaktors abzieht.

7. Verfahren nach Anspruch 6, wobei die Flussrate des aus dem Sumpf des Nachreaktors abgezogenen zweiten Stroms nach Maßgabe einer Temperatur geregelt wird, die an einer Stelle im Sumpf des Nachreaktors oder in einer aus dem Sumpf des Nachreaktors führenden Leitung gemessen wird.

8. Verfahren nach einem der vorhergehenden Ansprüche, umfassend eine Katalysatorbildungsstufe, wobei man eine wässrige Kobalt(II)-salzlösung mit Kohlenmonoxid und Wasserstoff unter Bildung des Kobaltkatalysators innig in Kontakt bringt.

9. Verfahren nach Anspruch 8, wobei die Bildung des Kobaltkatalysators, die Extraktion des Kobaltkatalysators in die organische Phase und die Hydroformylierung der Olefine einhergehend miteinander im selben Reaktor erfolgen, indem die wässrige Kobalt(II)-salzlösung, Kohlenmonoxid, Wasserstoff und Olefine sowie gegebenenfalls ein organisches Lösungsmittel im Reaktor unter Hydroformylierungsbedingungen innig in Kontakt gebracht werden.

10. Verfahren nach einem der vorhergehenden Ansprüche, wobei man den aus dem Reaktor oder Nachreaktor abgezogenen ersten Strom und zweiten Strom in Gegenwart von wässriger Kobalt(II)-salzlösung mit Sauerstoff behandelt, wobei der Kobaltkatalysator unter Bildung von Kobalt(II)-salzen zersetzt wird und diese in die wässrige Phase extrahiert werden; und man die Phasen anschließend trennt.

11. Verfahren nach Anspruch 10, wobei man einen Teil der wässrigen Phase in den Reaktor oder in die Katalysatorbildungsstufe zurückführt.

12. Verfahren nach einem der vorhergehenden Ansprüche, wobei der Reaktor ein vertikaler Rohrreaktor ist.

13. Verfahren nach einem der vorhergehenden Ansprüche, wobei der aus dem Reaktor bzw. Nachreaktor abgezogene zweite Strom 10 bis 80 Vol.-% wässrige Phase umfasst.

## Claims

1. A continuous process for hydroformylation of olefins having 6 to 20 carbon atoms in the presence of a cobalt catalyst in the presence of an aqueous phase with thorough mixing in a reactor wherein a hydroformylation products-containing first stream is withdrawn at the top of the reactor and an aqueous phase-containing second stream is withdrawn from the bottom of the reactor, which process comprises controlling the flow rate of the second stream in accordance with a temperature which is measured at a point in the bottom of the reactor or in a line leading out of the bottom of the reactor.

2. The process according to claim 1 wherein the flow rate of the second stream is controlled in accordance with the difference between a reaction temperature measured at at least one point of vigorous mixing in the reactor and the temperature measured at the point in the bottom of the reactor or in the line leading out of the bottom of the reactor.

3. The process according to claim 1 or 2 wherein the flow rate of the second stream is increased when the temperature difference increases.

4. The process according to any one of the preceding claims wherein the thorough mixing is carried out by means of a mixing nozzle.

5. The process according to any one of the preceding claims wherein the first stream and the second stream are passed into a post-reactor.

6. The process according to claim 5 wherein a hydroformylation products-containing first stream is withdrawn at the top of the post-reactor and an aqueous phase-containing second stream is withdrawn from the bottom of the post-reactor.

7. The process according to claim 6 wherein the flow rate of the second stream withdrawn from the bottom of the post-reactor is controlled in accordance with a temperature measured at a point in the bottom of the post-reactor or in a line leading out of the bottom of the post-reactor.

8. The process according to any one of the preceding claims, comprising a catalyst formation step wherein an aqueous cobalt(II) salt solution is brought into intimate contact with carbon monoxide and hydrogen to form the cobalt catalyst.

9. The process according to claim 8 wherein the formation of the cobalt catalyst, the extraction of the cobalt catalyst into the organic phase and the hydroformylation of the olefins are carried out in association with one another in the same reactor by bringing the aqueous cobalt(II) salt solution, carbon monoxide, hydrogen and olefins and, optionally, an organic solvent into intimate contact with one another in the reactor under hydroformylation conditions.

10. The process according to any one of the preceding claims wherein the first stream and second stream, withdrawn from the reactor or post-reactor, are subjected in the presence of aqueous cobalt(II) salt solution to oxygen treatment wherein the cobalt catalyst decomposes to form cobalt(II) salts which are extracted into the aqueous phase and the phases are then separated.

11. The process according to claim 10 wherein part of the aqueous phase is recycled into the reactor or into the catalyst formation step.

12. The process according to any one of the preceding claims wherein the reactor is a vertical tubular reactor.

13. The process according to any one of the preceding claims wherein the second stream, withdrawn from the reactor or post-reactor, comprises 10 to 80% by volume of aqueous phase.

## Revendications

1. Procédé continu d'hydroformylation d'oléfines contenant 6 à 20 atomes de carbone en présence d'un catalyseur de cobalt en présence d'une phase aqueuse par mélange dans un réacteur, un premier courant contenant des produits d'hydroformylation étant soutiré à la tête du réacteur et un second courant contenant une phase aqueuse étant soutiré du fond du réacteur, **caractérisé en ce que** le débit du second courant est ajusté en fonction d'une température qui est mesurée à un emplacement dans le fond du réacteur ou dans une conduite conduisant hors du fond.

2. Procédé selon la revendication 1, dans lequel le débit du second courant est ajusté en fonction de la différence entre une température de réaction mesurée à au moins un emplacement de mélange intensif dans le réacteur et la température mesurée à l'emplacement dans le fond du réacteur ou dans la conduite conduisant hors du fond.

3. Procédé selon la revendication 1 ou 2, dans lequel le débit du second courant est augmenté lorsque la différence de température augmente.

4. Procédé selon l'une quelconque des revendications précédentes, dans lequel le mélange a lieu au moyen d'une buse de mélange.

5. Procédé selon l'une quelconque des revendications précédentes, dans lequel le premier courant et le second courant sont conduits dans un réacteur secondaire.

6. Procédé selon la revendication 5, dans lequel un premier courant contenant des produits d'hydroformylation est soutiré à la tête du réacteur secondaire et un second courant contenant une phase aqueuse est soutiré au fond du réacteur secondaire.

7. Procédé selon la revendication 6, dans lequel le débit du second courant soutiré au fond du réacteur secondaire est ajusté en fonction d'une température qui est mesurée à un emplacement dans le fond du réacteur secondaire ou dans une conduite conduisant hors du fond du réacteur secondaire.

8. Procédé selon l'une quelconque des revendications précédentes, comprenant une étape de formation du catalyseur, selon laquelle une solution aqueuse de sels de cobalt (II) est mise en contact intime avec du monoxyde de carbone et de l'hydrogène pour former le catalyseur de cobalt.

9. Procédé selon la revendication 8, dans lequel la formation du catalyseur de cobalt, l'extraction du catalyseur de cobalt dans la phase organique et l'hydroformylation des oléfines ont lieu ensemble dans le même réacteur, par mise en contact intime de la solution aqueuse de sels de cobalt (II), de monoxyde de carbone, d'hydrogène et des oléfines, ainsi qu'éventuellement d'un solvant organique dans le réacteur dans des conditions d'hydroformylation.

10. Procédé selon l'une quelconque des revendications précédentes, dans lequel le premier courant et le second courant soutirés du réacteur ou du réacteur secondaire sont traités avec de l'oxygène en présence d'une solution aqueuse de sels de cobalt (II), le catalyseur de cobalt étant décomposé pour former des sels de cobalt (II) et ceux-ci étant extraits dans la phase aqueuse ; puis les phases étant séparées.

11. Procédé selon la revendication 10, dans lequel une partie de la phase aqueuse est recyclée dans le réacteur ou dans l'étape de formation du catalyseur.

12. Procédé selon l'une quelconque des revendications précédentes, dans lequel le réacteur est un réacteur tubulaire vertical.

13. Procédé selon l'une quelconque des revendications précédentes, dans lequel le second courant soutiré du réacteur ou du réacteur secondaire comprend 10 à 80 % en volume de phase aqueuse.
